Europäisches Patentamt

European Patent Office

Office européen des brevets

(19)

(11) Numéro de publication: **0 371 859**
**A1**

# (12) DEMANDE DE BREVET EUROPEEN

(21) Numéro de dépôt: 89403262.2

(22) Date de dépôt: **24.11.89**

(51) Int. Cl.5: **C07C 17/34, F28G 1/12**

(30) Priorité: **01.12.88 FR 8815758**

(43) Date de publication de la demande:
**06.06.90 Bulletin 90/23**

(84) Etats contractants désignés:
**AT BE CH DE ES FR GB GR IT LI LU NL SE**

(71) Demandeur: **ATOCHEM**
**4 & 8, Cours Michelet La Défense 10**
**F-92800 Puteaux(FR)**

(72) Inventeur: **Clair, René**
**La Vrignoise Saint-Julien**
**F-13500 Martigues(FR)**
Inventeur: **Correia, Yves**
**Les Lauzières**
**F-04160 Château-Arnoux(FR)**
Inventeur: **Demaizière, Claude**
**Les Esperelles**
**F-13500 Martigues(FR)**

(54) **Procédé de nettoyage de tubes.**

(57) L'invention concerne un procédé de nettoyage de tubes en fonctionnement dans un procédé. On introduit dans ces tubes des particules qui sont entraînées par le fluide circulant dans ces tubes, puis qu'on enlève dudit fluide ces particules. Elle est particulièrement utile pour décoker les fours de pyrolyse du dichloroéthane.

EP 0 371 859 A1

## PROCEDE DE NETTOYAGE DE TUBES

La présente invention concerne un procédé de nettoyage de tubes, et plus particulièrement les tubes encrassés par des dépôts solides pouvant être constitués essentiellement de coke.

Dans de nombreux procédés industriels mettant en oeuvre des hydrocarbures, il peut se produire des réactions chimiques dont le résultat est une précipitation de carbone, par exemple sous forme de coke. Dans la réaction de pyrolyse du 1,2-dichloroéthane (D 12) pour donner du chlorure de vinyle (VCM) et de l'acide chlorhydrique (HCl), il se produit à côté de cette réaction principale des décompositions conduisant à des dépôts de coke. Cette synthèse est décrite dans KIRK OTHMER, volume 23, pages 875-876, 3° édition. Le brevet US 4 324 932 décrit aussi des dépôts de coke au cours de la pyrolyse du D 12 en VCM et HCl. Les dépôts de coke conduisent à des pertes de productivité et de rendement obligeant à arrêter le four de cracking au bout de quelques mois pour enlever ces dépôts.

Le brevet US 4 297 147 décrit des décokages conventionnels et propose un décokage par des billes injectées dans les tubes et entraînées par un courant d'azote. Cette opération se fait à l'arrêt du four, c'est-à-dire que les tubes du four ne contiennent plus de fluide process.

Ces procédés ont l'inconvénient de nécessiter l'arrêt du four. Il existe aussi des procédés préventifs, par exemple le brevet européen EP 241020 dit qu'on peut réduire la formation de coke dans des tubes de cracking d'hydrocarbures à condition de les avoir mis en contact avec un mélange de Si-$(OC_2H_5)_4$ et de 2-éthylhexanoate d'étain. Ce système n'est pas complètement efficace et de plus certains de ces inhibiteurs peuvent être des poisons ou des catalyseurs indésirables.

On a maintenant trouvé un procédé beaucoup plus simple qui permet de nettoyer ou de décoker en marche, sans arrêter le procédé.

La présente invention est un procédé de nettoyage de tubes en fonctionnement dans un procédé, caractérisé en ce qu'on introduit dans ces tubes des particules qui sont entraînées par le fluide circulant dans ces tubes, puis qu'on enlève dudit fluide les particules.

Le procédé de l'invention permet d'enlever des dépôts sur une surface difficilement accessible. Les tubes de l'invention peuvent être des tubes d'un échangeur de chaleur, les tubes d'un four de cracking, des tuyaux de liaison entre différents appareils d'un procédé, c'est aussi la calandre d'un échangeur de chaleur, dans ce cas l'invention permet de nettoyer l'extérieur des tubes de l'échangeur. L'appellation "tubes" dans l'invention veut dire la surface intérieure d'une capacité contenant un fluide en circulation. Par exemple, dans le cas d'un échangeur sous forme de tubes à double enveloppe et pour un fluide circulant dans cette double enveloppe, l'invention permet le nettoyage de la surface intérieure du tube extérieur et de la surface extérieure du tube intérieur. Le nettoyage concerne l'enlèvement de tout dépôt sous quelque forme que ce soit. L'invention est particulièrement utile pour les dépôts très adhérents tels que du tartre ou du coke. L'invention s'applique avantageusement aux fours de cracking ou de pyrolyse, et plus particulièrement à la pyrolyse du D 12. De tels fours sont décrits dans les brevets EP 180925, EP 225617, EP 264065, EP 276775 et EP 270007.

L'invention s'applique pendant le fonctionnement, c'est-à-dire que le procédé n'est pas arrêté et donc la production n'est pas totalement interrompue comme dans l'art antérieur.

Les particules peuvent être quelconques pourvu qu'elles puissent être entraînées par le fluide et soient efficaces pour nettoyer les tubes. Il est aussi nécessaire de vérifier que les particules ne sont pas gênantes pour le procédé, c'est-à-dire ne catalysent pas des réactions parasites, indésirables ou dangereuses. Ces particules peuvent être en matériau quelconque, notamment alumine, silice, verre ou silico alumine, c'est par exemple du sable, des scories.

On peut aussi utiliser les particules métalliques telles que par exemple en acier, inox, nickel, inconel. Le matériau est choisi facilement par l'homme de métier en fonction des conditions du procédé, et notamment de la nature, pression et température du fluide. L'homme de métier doit aussi tenir compte de la dureté des particules, des dépôts à enlever et des tubes sur lesquels sont accrochés ces dépôts. On peut aussi utiliser un mélange de particules de différents matériaux. Avantageusement, pour nettoyer les fours de pyrolyse de D 12, on utilise des particules en acier ou en inox. De préférence les particules ont une surface spécifique très faible, c'est-à-dire qu'elles n'ont pas ou peu de pores.

On évite d'utiliser des particules ayant des arrêtes vives pour ne pas provoquer d'abrasion des tubes. Par exemple, on utilise des particules de forme essentiellement sphérique, c'est-à-dire que leur volume représente une partie de la sphère dans laquelle chaque particule est inscrite pouvant dépasser 5 % et aller jusqu'à 100 %. De préférence ces particules ont peu ou pas d'arêtes vives. La taille des particules est fonction de leur densité et de la vitesse, et de la densité du fluide puisqu'on pense que les particules agissent sur les dépôts essentiellement par l'intermédiaire de leur énergie cinétique et le nombre d'impacts. On peut aussi utiliser un mélange de particules de différentes formes.

Il est avantageux que la taille des particules, c'est-à-dire le diamètre de la sphère dans laquelle chaque particule s'inscrit, soit inférieur à 10 mm et de préférence compris entre 0,5 et 10 mm.

La répartition des particules selon leur taille est sans importance pourvu qu'elles soient toutes transportées par le fluide et récupérables ensuite, par exemple pour décoker des tubes on utilise des particules d'acier dont 80 % en nombre ont une taille comprise entre 2 et 4 mm.

L'introduction des particules peut se faire par tout moyen, il est commode d'avoir un bac en charge avec une vanne ou une écluse ou tout dispositif équivalent. On peut introduire en un ou plusieurs points sur le même tube, ou séparemment ou simultanément sur plusieurs tubes s'ils fonctionnent en parallèle. Il est préférable de faire l'introduction dans les conditions les plus simples, par exemple pour nettoyer des fours de pyrolyse, notamment de D 12, l'introduction se fait avant la zone de chauffage. Il est nécessaire ensuite d'enlever les particules qu'on a injecté dans le fluide du procédé.

Cette opération est conventionnelle et peut se faire à l'aide de filtres, ou de cyclones ou tout moyen équivalent. Le cas échéant, en même temps qu'on récupère les particules, on récupère aussi les matériaux qui constituaient le dépôt, tel que le coke.

Avantageusement on introduit une quantité de particules inférieure à 15 kg par Kg de fluide circulant à l'entrée des tubes à nettoyer, et de préférence comprise entre 0,01 et 10 kg/Kg, pour des vitesses de fluide circulant dans les tubes comprises entre 5 et 50 m/s.

Le procédé de l'invention est particulièrement utile pour nettoyer les fours de pyrolyse du D 12.

Le plus souvent ces fours sont alimentés en D 12 gazeux entre 1 et 30 bars, 100 à 300° C, la vitesse dans les tubes étant 5 à 30 m/s. En sortie du four la température peut atteindre jusqu'à 550° C et la vitesse 25 à 50 m/s.

Cette opération de nettoyage peut se faire en une ou plusieurs fois et à toute périodicité selon les nécessités du procédé. Par exemple, dans le cas du décokage, on peut faire plusieurs injections de particules de courte durée séparées d'un délai correspondant au moins au temps de séjour des particules et des débris dans le four, ceci pour éviter un décokage trop violent qui pourrait obstruer les tubes. On vérifie l'efficacité du décokage soit lors de la séparation des particules d'avec le fluide parce qu'on y retrouve des débris de coke, soit par la mesure des performances du procédé.

Selon le procédé, les produits mis en oeuvre, les contraintes, on effectue un nouveau décokage par exemple, quelques mois après. Mais on peut aussi ne pas attendre que les performances du procédé s'altèrent sensiblement et procéder à des nettoyages plus fréquents. C'est l'avantage essentiel du procédé de l'invention qui ne nécessite pas d'arrêt de la production.

Selon les industries dans lesquelles le procédé de l'invention s'applique, il est parfois nécessaire avant le nettoyage de modifier les conditions de fonctionnement de l'appareil dont il faut nettoyer les tubes, par exemple en réduisant le régime de production ou en diluant le fluide passant dans les tubes. Avantageusement, avant de nettoyer un four de pyrolyse de D 12 on baisse la température du four (sans l'arrêter), ou on dilue le D 12 à l'entrée par de l'HCl de façon qu'après le décokage qui donne toujours une augmentation de la cinétique de réaction on n'ait pas d'emballement de la réaction annulant en quelques heures les effets du décokage. Ce cracking en présence d'HCl est décrit dans le brevet européen 195719, au nom de la demanderesse.

Les exemples suivants illustrent l'invention.

EXEMPLE 1 (non conforme à l'invention)

Un four de cracking de 1,2-dichloroéthane (D12) à une seule passe formée par un tube de 350 m de longueur et 100 mm de diamètre intérieur sous forme de parties droites et de coudes autour desquelles sont disposées des bruleurs alimentés en gaz naturel, est alimenté par 21000 kg/h de D 12 à 200° C sous 14 bars effectifs. La température de sortie est de 500° C, le taux de cracking, c'est-à-dire la fraction molaire de D 12 transformée en CVM, est de 57 %.

La perte de charge du four (c'est-à-dire la différence de pression entre l'entrée et la sortie) passe de 2,5 bars à 5 bars en 4 mois. Ce qui oblige à augmenter la pression à l'entrée dans un premier temps, puis dans un deuxième temps à baisser la production jusqu'à ce qu'il soit nécessaire de décoker le four.

On arrête donc tous les 4 mois le four puis on enlève le coke soit par brulage à l'air, ce qui exige 64 heures d'arrêt total de la production, soit par un jet de billes d'acier poussées à l'azote, ce qui exige 32 heures d'arrêt total de la production.

EXEMPLE 2 (conforme à l'invention)

Dans un four identique à celui de l'exemple 1 et fonctionnant de la même façon, mais équipé (i) d'un bac en charge avec une vanne d'isolement à passage direct et dont l'atmosphère est reliée aussi au D 12 à l'entrée du four, et (ii) d'un cyclone en sortie communiquant en partie basse par un sas avec une capacité, on effectue tous les mois l'opération suivante :

En 1 heure on baisse le profil de la température, et donc la température de sortie de 500 à 460°C corrélativement le taux de cracking baisse à 40 %, puis on injecte 100 kg de billes d'acier (diamètre moyen 2,4 mm, 85 % des billes ayant un diamètre entre 2 et 2,8 mm) par batch de 1,25 Kg en 5 secondes toutes les 25 secondes, ce qui prend environ 30 minutes.

Les billes sont injectées par le bac en charge à l'entrée et récupérées dans la capacité en partie basse du cyclone en même temps que des particules noires qui sont des fragments de dépôts carbonés. A la fin de cette injection le taux de cracking est remonté de lui-même à 50 %, puis on monte progressivement le profil et la température de sortie (en agissant sur les bruleurs) pour retrouver puis maintenir un taux de cracking de 57 % pendant un mois.

On constate qu'en 1 mois entre 2 opérations, la perte de charge évolue de 2,5 à 3 bars. On peut donc maintenir une production de VCM sans arrêt complet pour décokage, il suffit de baisser la production pendant l'injection des billes et ce pendant moins de 2 heures.

EXEMPLE 3 (conforme à l'invention)

On opère comme dans l'exemple 2, mais avant l'injection des billes on ajoute 2500 Kg/h d'HCl aux 21000 Kg/h de D 12, ce qui évite de baisser le taux de cracking et donc de baisser la production. Il suffit d'un peu de pression ; alors qu'on avait atteint 3 bars avant de décider le nettoyage en marche, les 2500 Kg/h d'HCl font passer à 3,5 bars. Puis on fait l'injection un peu plus vite que dans l'exemple 2, à raison de batch de 2 Kg en 5 secondes, toutes les 20 secondes et ce pendant environ 20 minutes. Puis on baisse progressivement l'HCl à l'entrée tout en baissant le profil de température.

On continue ensuite d'opérer comme dans l'exemple 2 en augmentant peu à peu le profil de température jusqu'au nettoyage suivant. L'injection d'HCl évite la baisse préventive de production avant l'injection des billes.

**Revendications**

1. Procédé de nettoyage de tubes en fonctionnement dans un procédé caractérisé en ce qu'on introduit dans ces tubes des particules qui sont entraînées par le fluide circulant dans ces tubes, puis qu'on enlève dudit fluide ces particules.

2. Procédé selon la revendication 1 caractérisé en ce que les particules sont métalliques.

3. Procédé selon la revendication 1 ou 2 caractérisé en ce que les particules sont essentiellement sphériques.

4. Procédé selon l'une des revendications 1 à 3 caractérisé en ce que la taille des particules est inférieure à 10 mm et de préférence comprise entre 0,5 et 10 mm.

5. Procédé selon l'une des revendications 1 à 4 caractérisé en ce que la quantité des particules est comprise entre 0,01 et 10 kg/Kg de fluide circulant dans les tubes.

6. Procédé selon l'une des revendications 1 à 5 caractérisé en ce qu'on l'applique aux fours de pyrolyse du dichloroéthane.

Office européen
des brevets

# RAPPORT DE RECHERCHE EUROPEENNE

Numero de la demande

EP 89 40 3262

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | FR-A-2 291 469 (LESLIE CO.) * Page 1, lignes 1-3; page 2, lignes 23-32; page 4, figures 1-5 * | 1,2,3 | C 07 C 17/34 F 28 G 1/12 |
| Y | | 6 | |
| A | | 4,5 | |
| | --- | | |
| Y | FR-A-1 157 791 (SOCIETE BELGE DE L'AZOTE ET DES PRODUITS CHIMIQUES DU MARLY) * Page 5 * | 6 | |
| | --- | | |
| A,D | US-A-4 297 147 (NUNCIATO et al.) * Résumé * | 4,5 | |
| | ----- | | |

|  |
|---|
| **DOMAINES TECHNIQUES RECHERCHES (Int. Cl.5)** |
| B 08 B C 07 C F 28 G |

Le présent rapport a été établi pour toutes les revendications

| Lieu de la recherche | Date d'achèvement de la recherche | Examinateur |
|---|---|---|
| LA HAYE | 26-02-1990 | VOLLERING J.P.G. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
..............................................................................
& : membre de la même famille, document correspondant

EPO FORM 1503 03.82 (P0402)